Europäisches Patentamt

European Patent Office

Office européen des brevets

(19)

(11) Publication number : **0 489 538 A1**

(12) **EUROPEAN PATENT APPLICATION**

(21) Application number : 91310996.3

(22) Date of filing : 28.11.91

(51) Int. CI.⁵ : **A61N 5/06,** A61F 7/08, A61F 13/00

(30) Priority : 30.11.90 JP 337555/90

(43) Date of publication of application :
10.06.92 Bulletin 92/24

(84) Designated Contracting States :
AT BE CH DE DK ES FR GB GR IT LI LU NL SE

(71) Applicant : **TAKEDA CHEMICAL INDUSTRIES,
LTD.**
1-1, Doshomachi 4-chome
Chuo-ku, OSAKA (JP)

(71) Applicant : **TACHIBANA TEXTILE FABRICS,
CO., LTD.**
1225, Oaza-ogaito Kumatori-cho
Sen-nangun Osaka 590-04 (JP)

(72) Inventor : **Aki, Osami**
19-1, Daiwahigashi 5-chome
Kawanishi, Hyogo 666-01 (JP)
Inventor : **Kashi, Rikujiro**
3-202, 2 Naka-aoki 2-chome
Kawaguchi, Saitama 332 (JP)
Inventor : **Okamoto, Satsuki**
68, Shimokaiinji-yokoyama
Nagaokakyo, Kyoto 617 (JP)
Inventor : **Tachibana, Hideki**
595-1 Itahara
Izumiohtsu, Osaka 595 (JP)
Inventor : **Tanase, Setsumi**
399-5, Kuroda
Han-nan, Osaka 599-02 (JP)

(74) Representative : **Laredo, Jack Joseph
Elkington and Fife Prospect House 8
Pembroke Road
Sevenoaks, Kent TN13 1XR (GB)**

(54) **Articles having healthful and therapeutic effects and production of the same.**

(57) There is described an article which includes a coating of a composition provided at least at a part thereof, said composition comprising germanium, ceramics which radiate far infrared rays on heating, and a binder. Furthermore, the present invention provides a process for producing a therapeutic article which comprises coating a composition on a part of an article or a material for said article and heating said coated article or material, said composition comprising germanium, ceramics which radiate far infrared rays on heating, and a binder. The article may be used in clothing, bedclothes or furnishings.

Fig. 2

EP 0 489 538 A1

## FIELD OF THE INVENTION

The present invention relates to articles having healthful and therapeutic effects. More particularly, it relates to articles such as clothing and sheet materials, for example, various clothes, bedclothes, seat cloths and seat covers and the like which act to mitigate the condition of a stiff shoulder and fatigue, promote a good sleep to maintain good health and have a good effect on treating menstrual pain, knee pains, lumbago and physiological pains by being brought into contact with the human body, directly or indirectly.

## BACKGROUND OF THE INVENTION

It has been known that germanium has analgesic and anti-inflammatory activities for mitigating a stiff shoulder, lumbago, muscular pains and the like, and it has been used in therapeutic devices which are brought into contact with the skin. It is also known that ceramics which absorb heat energy by heating and radiate far infrared rays which are effective in raising the temperature in a deep area under the skin, and the action of the far infrared ray radiation causes microvascular dilation, promotion of blood circulation and metabolism, relaxation of sensory nerves or regulation of autonomic nerves.

The present inventors noted the effects of germanium and ceramics radiating far infrared rays by heating, and completed a skin contacting therapeutic device in the form of sheet in which both of them are combined and filed a patent application (JP-A 2-084966). During further intensive studies on such a therapeutic device, the present inventors have found that articles such as clothing, such as socks, underwear and bedclothes and the like as well as sheet materials such as seat cloths and seat covers and the like which are made by using a composition of germanium and ceramics having a certain composition, is useful for promoting health and especially useful as a therapeutic device. Thus, the present invention has been completed.

## OBJECTS OF THE INVENTION

The main object of the present invention is to provide articles having healthful and therapeutic effects.

This object as well as other objects and advantages of the present invention will become apparent to those skilled in the art from the following description with reference to the accompanying drawing.

## BRIEF EXPLANATION OF THE DRAWING

Fig. 1 is a graph illustrating the change of the body surface temperature as described in the Experiment 1 hereinafter.

Fig. 2 is a graph illustrating the change of temperature in a deep area under the skin as described in Experiment 2 hereinafter.

## SUMMARY OF THE INVENTION

According to the present invention, there is provided an article having healthful and therapeutic effects which comprises a coating of a composition provided at least at a part thereof, with said composition comprising germanium, ceramics which radiate far infrared rays by heating, and a binder. Further,, the present invention provides a process for producing an article having healthful and therapeutic effects which comprises coating a composition on a part of an article or a material for said article and heating said coated article or material, said composition comprising germanium, ceramics which radiate far infrared rays by heating, and a binder. In particular, the articles of the present invention are those which come into contact with the human body, preferably, those articles which come into contact with skin,directly or indirectly. Examples thereof include clothing such as socks, stockings, tights, underwear (e.g., skirt, slimmer, etc.), cloth for lining, a stomach band, a supporter, gloves, a wrist band, a hair band, ties, bedclothes (e.g., bed sheet, pillow cover, night clothes, etc.), blankets and the like as well as sheet materials such as seat cloths and seat covers and the like.

## DETAILED DESCRIPTION OF THE INVENTION

The germanium used in the composition of the present invention includes any material containing germanium such as inorganic germanium, organic germanium (e.g., an organic germanium complex containing, for example, 2 to 10 carbon atoms). Inorganic germanium having more than 99.9% purity is preferred.

The ceramics radiating far infrared rays (hereinafter referred to as far infrared ray radiation ceramics) are powder obtained by molding finely divided powder of $ZrO_2$, $MgO$, $Al_2O_3$, $SiO_2$, $Cr_2O_3$, $TiO_2$, $Fe_2O_3$ or the like,

or previously calcined powder thereof, alone or in combination, optionally sintering the molded material, and then pulverizing the molded material. The composition of the ceramics is not limited to a specific one so long as the ceramics radiate far infrared rays by heating with body temperature. Examples thereof include a powder mixture of $ZrO_2$, $SiO_2$ and $Al_2O_3$, a powder mixture of $ZrO_2$, $TiO_2$ and $Al_2O_3$, a powder mixture of $ZrO_2$, $MgO_2$ and $Fe_2O_3$, a powder mixture of $ZrO_2$, $SiO_2$, $Al_2O_3$ and $Fe_2O_3$, and a powder mixture of $SiO_2$, $Al_2O_3$ and $Fe_2O_3$ (this powder may be used without sintering). The wavelength of infrared rays to be radiated is preferably about 3 to 16 $\mu$, more preferably about 5 to 16 $\mu$ wherein a strong thermoresonance effect is obtained.

These germanium and ceramics preferably have average particle diameter of about 0.1 to 5 $\mu$, respectively, from the viewpoint of thermal effect. The weight ratio of germanium (as inorganic germanium) : ceramics in the composition is preferably 3 to 40 : 97 to 60. Usually, the composition contains the germanium in the amount of 0.45 to 20% by weight, preferably, 5 to 15% by weight, and ceramics in an amount of 9 to 30% by weight, preferably, 15 to 25% by weight.

As the binder used in the composition of the present invention, there can be used one or more liquid or paste binders. Preferred examples thereof include O/W type resin emulsions such as acrylate copolymer resin emulsion, ethylene-vinyl acetate copolymer resin emulsion, polyurethane resin emulsion, melamine resin emulsion, amine resin emulsion and the like. Usually, the composition contains the binder in an amount of 8 to 70% by weight, preferably, 10 to 45% by weight.

Optionally, the composition of the present invention can contain one or more additives such as a foaming agent, a crosslinking agent, a pigment and water.

As the foaming agent, any foaming agent can be used. In particular, it is preferred to use a foaming agent encapsulated in micro-capsules. Such an encapsulated foaming agent (hereinafter referred to as a micro-capsulated foaming agent) is commercially available and can be prepared by finely dividing a core material and covering the core material with a membrane material according to a known method such as a chemical method (e.g., interfacial polymerization, in situ polymerization, etc.), a physicochemical method (e.g., coacervation, interfacial precipitation, etc.) or a mechanical method (e.g., spray drying, etc.). Examples of the core material include hydrocarbons having low melting points such as propane, propylene, butane, isobutane, benzene, isopentane, hexane and the like. Examples of the membrane material include natural polymers such as gelatin-gum arabic, gelatin-carboxymethylcellulose and the like; synthetic polymers such as melamine resin, urea-formaldehyde resin, polyamide resin, epoxy resin, acrylate resin, vinyl chloride resin, polyurethane resin, polyurea resin, polystyrene resin and the like; lipid such as fats and oils, fatty acids and their derivatives and the like; and inorganic materials such as glass and the like. The composition can contain the foaming agent in an amount of up to 10% by weight, preferably, 3 to 8% by weight.

As the crosslinking agents to be used, there are melamine resin, urea resin, epoxy resin, ethyleneimine resin, isocyanate resin and the like. The composition can contain the crosslinking agent in an amount of up to 10% by weight, preferably, 3 to 8% by weight.

As the pigment, any suitable natural or synthetic organic or inorganic can be used to match the color of the composition with that of the article. The composition can contain the pigment in an amount of up to 12% by weight, preferably, up to 10% by weight.

The composition of the present invention may contain water to adjust the viscosity of the composition. Usually, the amount thereof is up to 10% by weight, preferably, up to 5% by weight.

The composition used in the present invention can be prepared by uniformly admixing these components according to a conventional method.

The article of the present invention can be in the form of clothing such as socks, stockings, tights, underwear, cloth for lining, a stomach band, a hair band, a supporter, gloves, a wrist band, ties, bedclothes, blankets and the like as well as sheet materials such as seat cloths, seat covers and the like which come into contact with the human body, directly or indirectly. The coating of the composition is provided at least at a part of the article so as to contact with the human body, directly or indirectly. Cloths and the like constituting the article may be conventional ones and the production of the article itself can be carried out by a conventional method. For example, cloths of natural fibers such as cotton, wool and the like, various synthetic fibers and mixed fibers are used to make the desired clothing or sheet material according to a conventional method. In addition, fibers which have antibacterial, deodorant or dampproof activity may be used alone or in combination. Examples of such fibers include antibacterial fibers wherein a metal such as stainless, titanium, copper or the like is spattered thereto; antibacterial, deodorant and dampproof fibers which are coated with zeolite particles; antibacterial and deodorant fibers impregnated with copper ion; and the like.

The coating can be provided to a predetermined part of the article or a material thereof such as the ankle, the arch of the foot or the like in the case of socks, the shoulder, the upper arm, bell, back and the like in the case of underwear. The coating can be-provided at not only the surface of the article but also provided to the inner part or back surface of the article. The coating can be provided by a conventional method such as print, and the like in a continuous pattern or a discontinuous pattern such as dot pattern or the like. Then, if necessary, after preliminary drying at 60 to 100°C for 2 seconds to 1 minute, the coating is heated at 120 to 250°C for 6 seconds to 1 minute to foam it. The preliminary heating and further allowing it to stand for drying can further improve resistance to washing. During the heating, the coating can be pressed by an aluminum plate and the like at a pressure of 2 to 8 kg/cm$^2$ and this can further improve handling and resistance to washing.

An article of the present invention acts to mitigate the condition of a stiff shoulder and fatigue, promotes a good sleep to maintain health and has a good effect in treating menstrual pain, knee pains, lumbago and physiological pains by being in contact with the human body, directly or indirectly.

The following Examples further illustrate the present invention in detail but are not to be construed to limit the scope thereof.

Example 1

| Components | % by weight |
|---|---|
| Micro-capsulated foaming agent (physicochemical method, isobutane-vinylidene chloride resin) | 6 |
| Ethylene-vinyl acetate copolymer resin emulsion (New Daifoam W-40NF manufactured by Dainichi Seika, Japan) | 34.5 |
| Acrylic ester copolymer resin emulsion (Seika Prene NF-10 manufactured by Dainichi Seika, Japan) | 13.5 |
| Ethyleneimine resin (Emafix TK manufactured by Dainichi Seika, Japan) | 6 |
| Pigment (black) | 5 |
| Far infrared ray radiation ceramics | 25 |
| Germanium | 10 |

These components were admixed uniformly. A dot pattern of 2 dots/cm$^2$ was printed on knit cloth by the mixture and, after preliminary drying at 70°C for 30 seconds, it was allowed to stand for 24 hours to naturally dry it. Then, an aluminum plate was placed on the printed surface and the cloth was heated at 200°C for 13 seconds with pressing at a pressure of 5 kg/cm$^2$. Double bottom socks for men were produced from the cloth thus obtained, wherein the printed surface of the cloth was provided inside thereof.

Example 2

| Components | % by weight |
|---|---|
| Micro-capsulated foaming agent as used in Example 1 | 6 |
| Ethylene-vinyl acetate copolymer resin emulsion (New Daifoam W-40NF manufactured by Dainichi Seika, Japan) | 18 |
| Acrylic ester copolymer resin emulsion (Seika Prene NF-10 manufactured by Dainichi Seika, Japan) | 24 |
| Ethyleneimine resin (Emafix TK manufactured by Dainichi Seika, Japan) | 7 |
| Pigment (white) | 10 |
| Far infrared ray radiation ceramics | 25 |
| Germanium | 10 |

These components were mixed uniformly. A dot pattern of 2.6 dots/cm$^2$ was printed on cotton 100% knit sheeting cloth by the mixture and, after preliminary drying at 70°C for 20 seconds, it was allowed to stand for 24 hours to naturally dry it. Then, an aluminum plate was placed on the printed surface and the cloth was heated at 200°C for 20 seconds with pressing at a pressure of 6 kg/cm$^2$. An underwear shirt for women was produced by the cloth thus obtained, wherein the printed surface of the cloth was provided inside thereof.

Example 3

| Components | % by weight |
|---|---|
| Micro-capsulated foaming agent as used in Example 1 | 6 |
| Ethylene-vinyl acetate copolymer resin emulsion (New Daifoam W-40NF manufactured by Dainichi Seika, Japan) | 26.5 |
| Acrylic ester copolymer resin emulsion (Seika Prene NF-10 manufactured by Dainichi Seika, Japan) | 11 |
| Ethyleneimine resin (Emafix TK manufactured by Dainichi Seika, Japan) | 6.5 |
| Pigment (white) | 10 |
| Far infrared ray radiation ceramics | 25 |
| Germanium | 10 |
| Water | 5 |

These components were admixed uniformly. A dot pattern was printed on the arch part of a cloth for ankle double fit socks of acrylic fiber 80%, nylon 15% and polyurethane 5% by the mixture. A flower dot pattern was also printed at the ankle part. In the socks, the thickness of the cloth at the arch part was 1/2 times that of the ankle part. Further, the dots at the arch should be convex, while the dots at the ankle should be flat. Then, the dot pattern was printed with the automatic printing device Model 5575 (manufactured by Mino Shoji K.K. in Japan) having a screen of 300 $\mu$m in thickness at the arch part and 80 $\mu$m in thickness at the ankle part. The dot pattern was 6 dots/cm$^2$, 2 mm in diameter of each dot at the arch and the flower pattern was 21 dots/cm$^2$, 1.1 mm in diameter of each flower at the ankle. After preliminary drying at 70°C for 30 seconds, it was allowed to stand for 24 hours to naturally dry it. Then, a Teflon (trademark of polytetrafluoroethylene) sheet was placed on the printed surface and the cloth was heated at 150°C for 10 seconds with pressing at a pressure of 3 kg/cm$^2$. Double fit socks for women were produced from the cloth thus obtained, wherein the printed surface of the cloth was provided inside thereof.

Example 4

| Components | % by weight |
|---|---|
| Micro-capsulated foaming agent as used in Example 1 | 6 |
| Ethylene-vinyl acetate copolymer resin emulsion (New Daifoam W-40NF manufactured by Dainichi Seika, Japan) | 29 |
| Acrylic ester copolymer resin emulsion (Seika Prene NF-10 manufactured by Dainichi Seika, Japan) | 24 |
| Ethyleneimine resin (Emafix TK manufactured by Dainichi Seika, Japan) | 3 |
| Far infrared ray radiation ceramics | 25 |
| Germanium | 10 |
| Water | 3 |

These components were admixed uniformly. A dot pattern of 2 dots/cm$^2$ was printed on the surface of a pile fabric of cotton 58%, acrylic fiber 22%, nylon 15% and polyurethane 5% by the mixture. After preliminary drying at 70°C for 40 seconds, it was allowed to stand for 24 hours to naturally dry it. Then, a Teflon sheet was placed on the printed surface and the cloth was heated at 180°C for 8 seconds with pressing at a pressure of 5 kg/cm$^2$. Men's socks for golf and tennis were produced from the cloth thus obtained, wherein the printed pile surface of the cloth was provided inside thereof.

Example 5

| Components | % by weight |
|---|---|
| Micro-capsulated foaming agent as used in Example 1 | 6 |
| Ethylene-vinyl acetate copolymer resin emulsion (New Daifoam W-40NF manufactured by Dainichi Seika, Japan) | 31.5 |
| Acrylic ester copolymer resin emulsion (Seika Prene NF-10 manufactured by Dainichi Seika, Japan) | 12.5 |
| Ethyleneimine resin (Emafix TK manufactured by Dainichi Seika, Japan) | 5 |
| Pigment (white) | 10 |
| Far infrared ray radiation ceramics | 25 |
| Germanium | 10 |

These components were admixed uniformly. A dot flower pattern of 17 dots/cm$^2$ was printed on a cloth for tights of 50 denier nylon and polyurethane. After preliminary drying at 60°C for 20 seconds, it was allowed to stand for 24 hours to naturally dry it. Then, a Teflon sheet was placed on the printed surface and the cloth was heated at 120°C for 20 seconds with pressing at a pressure of 3 kg/cm$^2$. Tights were produced from the cloth thus obtained, wherein the printed surface of the cloth was provided inside thereof to warm the legs.

Example 6

| Components | % by weight |
|---|---|
| Micro-capsulated foaming agent as used in Example 1 | 6 |
| Ethylene-vinyl acetate copolymer resin emulsion (New Daifoam W-40NF manufactured by Dainichi Seika, Japan) | 23.5 |
| Acrylic ester copolymer resin emulsion (Seika Prene NF-10 manufactured by Dainichi Seika, Japan) | 19.5 |
| Ethyleneimine resin (Emafix TK manufactured by Dainichi Seika, Japan) | 8 |
| Far infrared ray radiation ceramics | 25 |
| Germanium | 10 |
| Water | 8 |

These components were admixed uniformly. A cross stitching dot pattern of 21 dots/cm$^2$ having 1.1 mm in diameter of each dot was printed on moquette cloth of 100% acrylic fiber by the mixture. After preliminary drying at 60°C for 30 seconds, it was allowed to stand for 24 hours to naturally dry it. Then, a Teflon sheet was placed on the printed surface and the cloth was heated at 120°C for 18 seconds with pressing at a pressure of 3 kg/cm$^2$. A seat cloth of a car was produced from the cloth thus obtained, wherein the printed surface of the cloth was provided outside of the cloth at the position faced to the back and hip parts of the human being. Likewise, a seat cloth was produced by providing the printed surface on the inside (back surface) thereof.

Example 7

| Components | % by weight |
|---|---|
| Micro-capsulated foaming agent as used in Example 1 | 6 |
| Ethylene-vinyl acetate copolymer resin emulsion (New Daifoam W-40NF manufactured by Dainichi Seika, Japan) | 33.7 |
| Acrylic ester copolymer resin emulsion (Seika Prene NF-10 manufactured by Dainichi Seika, Japan) | 13.3 |
| Ethyleneimine resin (Emafix TK manufactured by Dainichi Seika, Japan) | 8 |
| Far infrared ray radiation ceramics | 25 |
| Germanium | 10 |
| Water | 4 |

These components were mixed uniformly. A dot pattern of 4 dots/cm$^2$ having 2 mm in diameter of each dot was printed on the inner surface of double tubular knitted fabric for a supporter composed of 30% of Egyptian cotton, 20% of nylon, 35% of polyurethane and 15% of rayon by the mixture and, after preliminary drying at 70°C for 40 seconds, it was allowed to stand for 24 hours to naturally dry it. Then, a Teflon sheet was placed on the printed surface and the cloth was heated at 180°C for 8 seconds with pressing at a pressure of 3 kg/cm$^2$. A supporter for warming a knee, an elbow or the like was produced by the fabric thus obtained, wherein the printed surface of the fabric was provided inside thereof.

In order to show the thermal effect of an article of the present invention, the following experiments were done.

Experiment 1

A seat of a car was made from the following seat cloth.

Cloth 1: The seat cloth as produced in Example 6 which had the printed surface provided on the outside of the cloth at the position faced to the back and hip as produced.

Cloth 2: The seat cloth as produced in Example 6 which had the printed surface provided on the inside of the cloth at the position corresponding to the back and hip parts.

Cloth 3: A seat cloth of moquette cloth having no printed surface (control).

The person put on an undershirt, a shirt and a jacket of wool and sat on the seat of each seat cloth for 30 minutes. Then, the person rose from the seat and put out all the wear. The change of the body surface temperature with time was recorded.

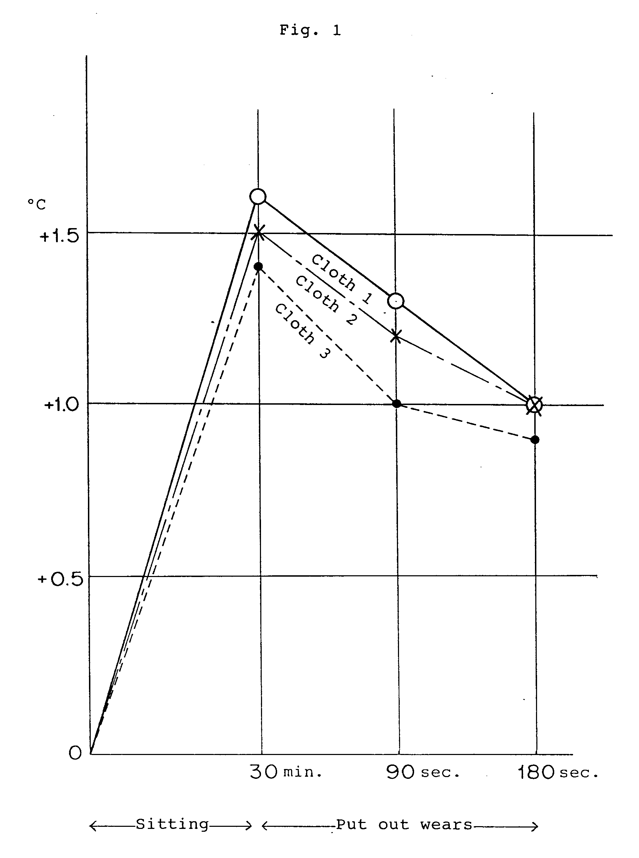

The results are shown in Fig. 1. In Fig. 1, the solid line represents Cloth 1, the chain line represents Cloth 2 and the dotted line represents Cloth 3 (control). As can be seen from the results of Fig. 1, when the seat cloth having the printed surface according to the present invention is used, a higher body temperature is maintained even after rising from the seat. This shows a better thermal effect than compared with the control cloth.

Experiment 2

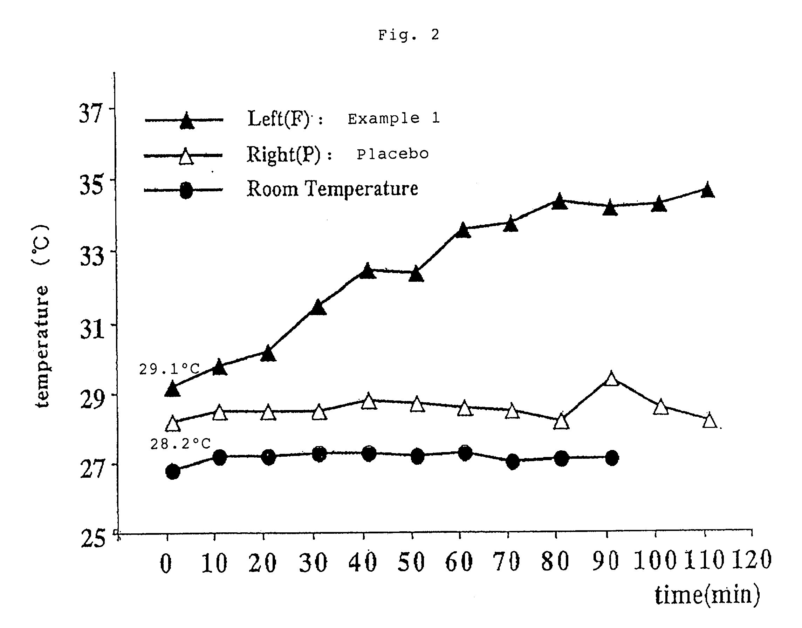

The temperature of the body tissue at 1 cm in depth of the sole of each foot of a person who had the sock produced in Example 1 on the left foot and had a placebo sock on the right foot of the same size and color as that of the sock of Example 1, was measured with a heat flow compensation type thermometer for a deep area (Coretemp, CTM-204, Terumo, Japan) at 10 minutes intervals after about 20 to 30 minutes of a stabilizing period. The results are shown in Fig. 2. As can be seen from Fig. 2, the socks of Example 1 have a better thermal effect than the placebo socks.

As described hereinabove, according to the present invention, there is provided an article which mitigates the condition of a stiff shoulder and fatigue, promotes a good sleep to maintain good health and has a good effect in treating knee pains, lumbago and physiological pains by being in contact with the human body directly or indirectly due to an electrical- and thermotherapeutical effect of germanium and far infrared ray radiation ceramics.

**Claims**

1. An article which comprises a coating of a composition provided at least at a part thereof, said composition comprising germanium, ceramics which radiate far infrared rays by heating, and a binder.

2. The article of claim 1 which is selected from socks, underwear, tights, a seat cover, a seat cloth, a stomach band, a supporter, and bedclothes.

3. The article of claim 1, wherein said composition comprises 0.45 to 20% by weight of germanium as inorganic germanium, 9 to 30% by weight of ceramics and 8 to 70% by weight of a binder.

4. A process for producing an article which comprises coating a composition on a part of an article or a material for said article and heating said coated article or material, said composition comprising germanium, ceramics which radiate far infrared rays by heating, and a binder.

5. A process of claim 4, wherein preliminary heating is carried out before heating.

6. A process of claim 5, wherein the preliminary heated composition is allowed to stand at room temperature before heating.

7. A process of claim 4, wherein said composition comprises 0.45 to 20% by weight of germanium as inorganic germanium, 9 to 30% by weight of ceramics and 8 to 70% by weight of a binder.

Fig. 1

Fig. 2

EP 0 489 538 A1

13

European Patent
Office

**EUROPEAN SEARCH REPORT**

Application Number

EP    91 31 0996

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 347 197 (TAKEDA CHEMICAL INDUSTRIES) | 1-4,7 | A61N5/06 |
| A | * the whole document * | 5-6 | A61F7/08 |
| | --- | | A61F13/00 |
| A | WORLD PATENTS INDEX LATEST Week 8645, Derwent Publications Ltd., London, GB; AN 86-295163 & JP-A-61 217 173 (IMAMURA) 26 September 1986 * abstract * | 1-7 | |
| | --- | | |
| A | EP-A-0 348 093 (TAKEDA CHEMICAL INDUSTRIES) * abstract * | 1 | |
| | --- | | |
| A | WORLD PATENTS INDEX LATEST Week 8813, Derwent Publications Ltd., London, GB; AN 88-087891 & JP-A-63 038 471 (NEX) 19 February 1988 * abstract * | 1 | |
| | ----- | | **TECHNICAL FIELDS SEARCHED (Int. Cl.5)** A61N A61F |

The present search report has been drawn up for all claims

| Place of search | Date of completion of the search | Examiner |
|---|---|---|
| THE HAGUE | 10 MARCH 1992 | NICE P. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
 
& : member of the same patent family, corresponding
   document

EPO FORM 1503 03.82 (P0401)